(19) **Europäisches Patentamt**

**European Patent Office**

**Office européen des brevets**

(11) **EP 0 890 366 B1**

(12) **EUROPÄISCHE PATENTSCHRIFT**

(45) Veröffentlichungstag und Bekanntmachung des Hinweises auf die Patenterteilung:
**03.09.2003 Patentblatt 2003/36**

(51) Int Cl.[7]: **A61L 15/58**, A61F 13/02

(21) Anmeldenummer: **98111570.2**

(22) Anmeldetag: **24.06.1998**

(54) **Trägermaterial für medizinische Zwecke**

Carrier for medical uses

Support à usage médical

(84) Benannte Vertragsstaaten:
**AT DE ES FR GB IT NL SE**

(30) Priorität: **12.07.1997 DE 19729905**

(43) Veröffentlichungstag der Anmeldung:
**13.01.1999 Patentblatt 1999/02**

(73) Patentinhaber: **Beiersdorf Aktiengesellschaft 20245 Hamburg (DE)**

(72) Erfinder:
- **Himmelsbach, Peter
  21614 Buxtehude (DE)**
- **Bodenschatz, Stefan, Dr.
  21614 Buxtehude (DE)**

(56) Entgegenhaltungen:
DE-A- 4 237 252    DE-A- 19 531 291
DE-A- 19 631 422    US-A- 4 323 061

**Beschreibung**

Die Erfindung betrifft ein Trägermaterial für medizinische Zwecke, vorzugsweise orthopädische Binden, Bandagen, welches wenigstens einseitig und zumindest partiell mit einer Heißschmelzklebemasse beschichtet ist.

Als Trägermaterialien für diese Zwecke sind bereits zahlreiche Materialien auf Folien-, Gewebe, Gewirke, Vlies-, Gel- oder Schaumstoff-Basis bekannt und werden auch in der Praxis eingesetzt. Die Materialien, welche oft noch mit einer Selbstklebemasse beschichtet sind, müssen hautverträglich, in der Regel Luft- und wasserdampfdurchlässig sowie gut anmodellierbar und anschmiegsam sein. Aufgrund dieser Anforderungen wird häufig ein möglichst dünner oder weicher Träger bevorzugt. Zur Handhabung und beim Gebrauch ist bei den Trägermaterialien aber auch eine ausreichende Festigkeit und gegebenenfalls begrenzte Dehnbarkeit gefordert. Weiterhin sollte das Trägermaterial auch nach dem Durchnässen eine ausreichende Festigkeit und geringe Dehnbarkeit aufweisen.

Dünne Träger, insbesondere aus Vliesen, sind gut luft- und wasserdampfdurchlässig. Für bestimmte Anwendungen ist jedoch ihre Festigkeit zu gering und ihre Dehnung zu hoch.

Für spezielle Anwendungen, zum Beispiel Tapes für funktionelle Tapeverbände zur Prophylaxe und Therapie von Verletzungen, Erkrankungen und Veränderungen am Bewegungsapparat, sind unelastische Träger mit einer hohen Festigkeit in Beanspruchungsrichtung erforderlich. Dies wird erreicht, indem Gewebe, üblicherweise aus Baumwolle oder Viskose, eingesetzt werden. In der Regel sind solche Trägermaterialien, mit entsprechend hohem Flächengewicht, kostenintensiv. Eine hohe Flexibilität ist nur durch ein Gewebe mit geringerer Festigkeit zu erreichen. Dieses weist im allgemeinen aber dann unter Beanspruchung eine gewisse Dehnung auf, welche für die Anwendung unerwünscht ist.

Generell können beim Durchnässen diese Verbände an Festigkeit verlieren oder stärker dehnbar sein. Dieses ist ebenfalls für die Anwendung unerwünscht und wird bisher durch ein häufigeres Verbandswechseln, welches auf der anderen Seite kostenintensiv ist, kompensiert.

Bekannt ist weiterhin im Stand der Technik das Laminieren mit Verstärkungsfäden durch die Reichspatentschrift 571 244, wobei die dort eingesetzten Verstärkungsfäden aber nicht hochfest sind. Dann ist der Schrift allgemein kein Hinweis auf einen unelastischen Träger zu entnehmen.

Weiter beschreibt AU 73555/74 beispielhaft ein mit Glasfäden verstärktes Trägermaterial für medikale Anwendungen auf Schäumbasis. Das hier beschriebene Trägermaterial ist aber elastisch oder zu mindestens plastisch verformbar.

US 4,668,563 beschreibt ein mit Glasfaser verstärktes Material, welches jedoch elastisch ist.

Weiter sind Verstärkungen auf dem Packmittelsektor nicht unbekannt.

Stark klebende orthopädische Bandagen und andere medizinische Produkte werden üblicherweise vollflächig mit einer Zink-Kautschuk-Klebemasse beschichtet. Das Verkleben derartiger Produkte auf der Haut zeigt nach dem Ablösen deutliche Hautirritationen und eine mechanische Beanspruchung der Haut. Die Verklebung läßt sich ohne Hilfsmittel nur unter Schmerzen lösen.

Fallweise kommt es zu allergischen Reaktionen. Die verwendeten Klebemassen führen darüber hinaus oft zu einem Massetransfer, d.h. einem Umspulen, auf die Haut.

Die Verwendung von hautfreundlichen Klebemassen, wie Acrylatklebemassen, ist aufgrund der geringen Scherstabilität und Anfaßklebrigkeit nicht erwägenswert. Eine Verbesserung durch eine Nachbehandlung, insbesondere Vernetzung, ist möglich, jedoch bleibt das Ergebnis insgesamt unbefriedigend. Weiterhin reicht die Klebkraft auf der Trägerrückseite solcher Systeme bei zirkulär angelegten Verbänden mit mehreren Lagen nicht für einen stabilen funktionalen Verband aus. Die propriorezeptive Wirkung ist gegenüber den Systemen mit einer Zink-Kautschuk-Klebemasse geringer.

Andere bekannte Klebesysteme auf Basis von herkömmlichen Blockcopolymeren sind zum einen durch hohen Stabilisatorzusatz nicht hautfreundlich beziehungsweise zeigen durch die hohe Kohäsivität bislang nur eine Eignung für technische Anwendungsfälle, zum anderen sind sie nicht stark hautklebend und hauthaftend einzustellen.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100%-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Haftschmelzklebemassen sind im Stande der Technik bereits vorbeschrieben. Sie basieren auf natürlichen oder synthetischen Kautschuken und/oder anderen synthetischen Polymeren.

Aufgrund ihrer hohen Härte ist für solche 100%-Systeme die Hauthaftung problematisch.

Vorteilhaft an den 100%-Systemen ist, daß bei ihnen verfahrenstechnisch ein Entfernen der Trägermatrix, d. h. der Hilfsmittel vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Es ist femer bekannt, derartige Selbstklebemassen nicht nur vollflächig sondern auch rasterpunktförmig, beispielsweise durch Siebdruck (DE P 42 37 252 C), wobei die Klebstoffpünktchen auch unterschiedlich groß und/oder unterschiedlich verteilt sein können (EP 0 353 972 B), oder durch Tiefdruck von in Längs- und Querrichtung zusammenhängenden Stegen aufzubringen (DE P 43 08 649 C).

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch darin, daß bei zu hoher Flächendeckung der an sich undurchlässigen Klebeschicht die Luft- und Wasserdampfdurchlässigkeit sich entsprechend verringert sowie der Klebemassenverbrauch steigt und bei geringer Flächendeckung der Klebeschicht die Klebeeigenschaften leiden, d.h., das Produkt löst sich, insbesondere bei schweren, textilen Trägermaterialien, zu leicht vom Untergrund.

Dokument DE-A-195 31 291 zeigt die Merkmale der Präaembel des Anspruchs 1.

Aufgabe der Erfindung war es deshalb, ein Trägermaterial zu entwickeln, welches die Anforderungen an Zugfestigkeit sowie Reißdehnung und Dauerbelastbarkeit erfüllt und welches zudem die im Stand der Technik beschriebenen Nachteile herkömmlicher Klebesysteme vermeidet.

Gelöst wird diese Aufgabe durch die Merkmale des Anspruchs 1.

Bevorzugte Ausführungsbeispiele sind in den Unterensprüchen beensprüchen Das Trägermaterial weist vorzugsweise eine Dehnung von weniger als 10% bei einer Belastung von 10 N/cm auf sowie vorzugsweise ein Flächengewicht von weniger als 350 g/m$^2$, bevorzugt weniger 200 g/m$^2$.

Weiter vorzugsweise zeigt das Trägermaterial eine Höchstzugkraftdehnung unter 25%, bevorzugt unter 15%, besonders bevorzugt unter 10%.

Das Trägermaterial kann mit einem Faden oder mehreren Fäden aus Monofil, Multifil, Stapelfasergarn oder Spinnfasergam und/oder mit orientierten hochfesten Fasern verstärkt sein.

Weiter können auch Zwirne oder Mischzwirne, insbesondere Siromischzwime, eingesetzt werden. Für spezielle Anwendung können auch Mischfaserfäden, -garne oder - zwirne eingesetzt werden. Hierbei kann es sich zum Beispiel um Umwindegarne oder spezielle Stapelfaserumwindegarne handeln.

Vorteilhaft ist hier, daß durch die Kombination von hochfesten Verstärkungen und Basismaterialien besonderen oder spezielle Eigenschaften schon in dem Verstärkungsfaden erreicht werden kann. Beispielhaft sind hier die Kombinationen von Glas oder Kohlenstoff und Baum- oder Zellwolle.

Die Fasern oder Fäden können dabei aus organischen oder anorganischen Materialien bestehen, so beispielsweise und bevorzugt aus Glas, Kohlenstoff (Carbon) oder speziellen Polyamiden bestehen, weiterhin können die Verstärkungsfasem wenigstens teilweise gefärbt sein, um das Trägermaterial optisch ansprechender zu gestalten. Auf diese Art ist es problemlos möglich, die verstärkten Träger optisch zu differenzieren. Hierzu bieten sich insbesondere gefärbte Glas- oder Polymerfäden an.

Vorteilhafterweise ist die Orientierung der Verstärkungsfäden oder -fasern entsprechend der Beanspruchung des Trägermaterials im Gebrauch ausgerichtet.

Das Trägermaterial ist weiter vorzugsweise mit den Fäden und/oder hochfesten Fasern laminiert. Die Fäden und/oder die hochfesten Fasern sollten fest mit dem Trägermaterial verbunden sein. Dies kann durch direktes Einarbeiten oder Einlassen der Fasern, Fäden oder Zwirne beziehungsweise Mischzwirne in den Träger geschehen, wie Einweben bei Geweben, Einstricken bei Gewirken, Einbetten beziehungsweise Einfügen beim Herstellungsverfahren von Folien, Gelen oder Schaumstoffen und Vliesen.

Die Fasern oder die hochfesten Fäden können aber auch nachträglich mit dem Träger verbunden werden, beispielsweise sind das Verschweißen oder Auflaminieren mit einer entsprechenden Verbindungsschicht genannt. Das Einlegen in die Klebmassenschicht bietet sich unter anderem hierzu an.

Bei einer vorteilhaften Ausführungsvariante zeigt das Trägermaterial durch den Zusatz von hochfesten Fasern oder Fäden mit einer Höchstzugkraft über 60 cN/tex eine Höchstzugkraft über 50 N/cm und eine Höchstzugkraftdehnung unter 25% bei einem Flächengewicht von unter 140 g/m$^2$. Ein solches Trägermaterial ist insbesondere geeignet, um als Träger für ein Tape-Band zu dienen.

Das Trägermaterial läßt sich weiter vorzugsweise bei einer Belastung von 30N/cm nach 50 Zyklen weniger als 20%, bevorzugt 10%, besonders bevorzugt weniger als 5%, verformen.

Das Trägermaterial ist weiter vorzugsweise von Hand senkrecht zur Orientierung der Verstärkung und/oder in Richtung der Verstärkung reißbar.

Darüber hinaus kann das Trägermaterial auch vorbehandelt sein.

Die Anzahl der an- oder eingebrachten Fäden beziehungsweise hochfesten Fasern hängt in erster Linie vom jeweils vorgesehenen Verwendungszweck und der angestrebten Höchstzugkraft sowie Höchstzugkraftdehnung des Trägermaterials, seiner eigenen Beschaffenheit und der jeweiligen Festigkeit der Fasern und Fäden selbst ab und kann deshalb in relativ weiten Grenzen variieren.

Mit steigender Verstärkung hält der Träger einer größeren Beanspruchung und Belastung stand. Auch sehr stark verstärkte Trägermaterialien sind in der Lage, große Mengen an Feuchtigkeit aufzunehmen oder durchzulassen, und üben somit eine angenehme Anwenderempfindung aus.

Weiter werden die Verstärkungen vorzugsweise gezielt entsprechend der Beanspruchungsrichtung des Trägermaterials eingefügt, d.h. in Längsrichtung. Sie können jedoch auch, wenn dies zweckdienlicher ist, zusätzlich oder nur in Quer- oder Schrägrichtung oder beispielsweise kurven-, spiral- oder zick-zackförmig oder regellos verlaufen. Dabei kann es wünschenswert und erreichbar sein, daß das Trägermaterial senkrecht zur Orientierung der Verstärkung und/oder in Richtung der Orientierung von Hand einreißbar ist.

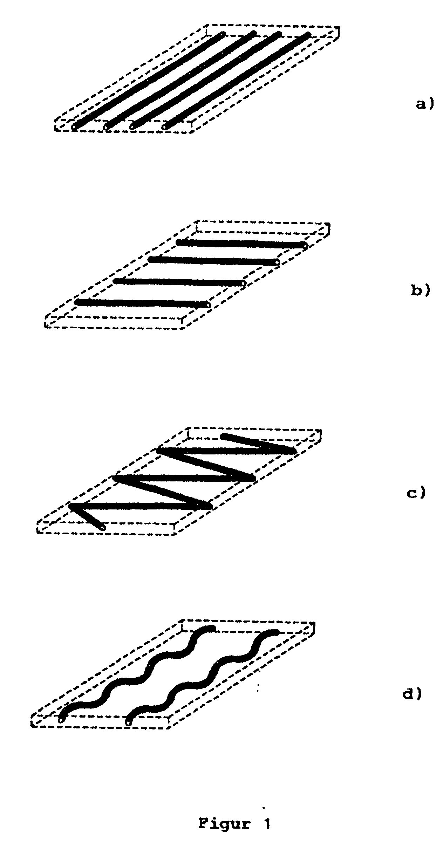

Die Figur 1 a - d zeigt beispielhaft verschiedene Anordnungsmöglichkeiten der Verstärkungsfäden in Längs-, Quer-, Zickzack- und Wellenform.

Bei den sogenannten Fertigtapeverbänden werden die Verstärkungsfäden oder -fasern bevorzugt gleich entsprechend der Beanspruchungsrichtung im angelegten Zustand angeordnet. Da diese Verbände schon fertig zugeschnitten oder ausgestanzt sind, ist eine Reißbarkeit hier nicht gefordert.

Als Trägermaterial für einen funktionellen Tapeverband kann beispielsweise ein flexibles Trägergewebe aus Baumwolle verwendet werden, dem Kohlenstoff (Carbon)-oder Glasfaserfäden in der Kette zugesetzt sind. So kann jeder 2te bis 12te Kettfaden aus dem hochfesten Material bestehen. Durch diese Konstruktion bleibt der Träger flexibel und läßt sich gut anmodellieren. Er hat dabei eine hohe Reißfestigkeit und seine Dehnbarkeit in Beanspruchungsrichtung ist deutlich verringert. Aufgrund der Sprödigkeit der hochfesten Fäden bleibt das Gewebe von Hand reißbar.

Ein zum Tapen geeigneter derartiger Träger weist beispielsweise auf eine Höchstzugkraft von 65 N/cm und eine Höchstzugkraftdehnung unter 10% bei einem Flächengewicht von 200 g/m$^2$.

Bei einer anderen möglichen Ausführungsform besteht das Gewebe aus 100% hochfesten Materialien in der Kette und ergibt Tapeträger mit besonders hoher Höchstzugkraft (>250 N/cm) und geringer Höchstzugkraftdehnung (<5%). Solch ein Träger wird für besonders hohe Belastungen und Beanspruchungen im Tapebereich verwendet.

Besonders vorteilhaft zeigte sich die Belastbarkeit im zyklischen Dauerstreßtest. Hier zeigte sich schon bei einer geringen Belastung von ca. 30 N/cm und wenigen Zyklen, daß die Dehnung eines handelsüblichen Trägermaterials gegenüber der Dehnung eines mit hochfesten Fasern oder Fäden verstärkten Trägermaterial ca. doppelt so hoch ist. Fallweise war die Dehnung sieben- bis achtfach höher als die der verstärkten Trägermaterialien, obwohl die Höchstzugkraft der Trägermaterialien vergleichbar war.

Nach einer Dauerstreßbelastung von 50 Zyklen weist ein erfindungsgemäße Träger je nach Materialzusammenstellung eine Verformung in Belastungsrichtung von weniger als 20 %, bevorzugt weniger als 10 %, besonders bevorzugt weniger als 5 %, auf.

Für das erfindungsgemäße Trägermaterial für medizinische Zwecke kommt weiter vorzugsweise eine Haftschmelzklebemasse zum Einsatz, die eine dynamisch-komplexe Glasübergangstemperatur bei einer Frequenz von 0,1 rad/s bevorzugt von -6 °C bis -30 °C, besonders bevorzugt von -9 °C bis -25 °C, aufweist.

Die Heißschmelzklebemasse kann dabei auf einer Blockcopolymerbasis aufgebaut sein, insbesondere A-B- oder A-B-A-Blockcopolymere oder deren Mischungen, wobei Phase A vornehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind. Polystyrolblöcke können aber auch in der Phase B enthalten sein, und zwar bis zu 20 Gew.-%.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

Der gesamte Styrolanteil im Polymer sollte weiter vorzugsweise niedriger sein als 35 Gew.-%, bevorzugt 5 Gew.-% bis 30 Gew.-%. Ein niedrigerer Styrolanteil macht die Haftschmelzklebemasse anschmiegsamer.

In einer vorteilhaften Ausführungsform setzt sich die Heißschmelzklebemasse wie folgt zusammen:

- 10 Gew.-% bis 90 Gew.-% Blockcopolymere
- 5 Gew.-% bis 80 Gew.-% Klebrigmacher wie Öle, Wachse, Harze und deren Mischungen, bevorzugt Mischungen aus Harzen und Ölen, wobei die eingesetzten Öle, Wachse und Harze vorzugsweise Kohlenwasserstofföle, -wachse und -harze sind
- weniger als 60 Gew.-% Weichmacher,
- weniger als 15 Gew.-% Additive
- weniger als 5 Gew.-% Stabilisatoren

Die vorzugsweise verwendeten Kohlenwasserstofföle (beispielsweise Paraffinkohlenwasserstofföle), -wachse und -harze dienen als Klebrigmacher, wobei sich diese durch ihre Konsistenz günstig auf die Hautverklebung auswirken.

Als Weichmacher finden langkettige Fettsäuren und/oder deren Ester Verwendung. Diese Zusätze dienen dabei der Einstellung der Klebeeigenschaften und der Stabilität.

Die Heißschmelzklebemasse weist einen Erweichungspunkt oberhalb von 70 °C, bevorzugt 85 °C bis 140 °C, auf.

Weiter vorzugsweise wird die Heißschmelzklebemasse durch Rasterdruck, Thermosiebdruck oder Tiefdruck auf das Trägermaterial aufgebracht, darüber hinaus in einer besonders bevorzugten Ausführung in Form von polygeometrischen Kalotten.

Der prozentuale Anteil, der mit der Heißschmelzklebemasse beschichteten Fläche sollte bei der partiellen Beschichtung des Trägermaterials mindestens 20% betragen und kann bis zu ca. 95% reichen, für spezielle Produkte bevorzugt 40% bis 60% sowie 70% bis 95%. Dieses kann gegebenenfalls durch Mehrfachapplikation erreicht werden, wobei gegebenenfalls auch Heißschmelzklebemassen mit unterschiedlichen Eigenschaften eingesetzt werden können.

Weiter vorzugsweise wird die Heißschmelzklebemasse mit einem Flächengewicht von größer 15 g/m$^2$, bevorzugt zwischen 70 g/m$^2$ und 300 g/m$^2$, ganz besonders bevorzugt zwischen 90 g/m$^2$ und 160 g/m$^2$, auf das Trägermaterial beschichtet.

Weiter vorzugsweise weist das beschichtete Trägermaterial auf eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), und/oder eine Wasserdampfdurchlässigkeit von größer 500 g/(m$^2$*24h), bevorzugt größer 1000 g/(m$^2$*24h), ganz besonders bevorzugt größer 2000 g/(m$^2$*24h).

Weiterhin können der Heißschmelzklebemasse nichtklebende Substanzen, wie beispielsweise mineralische Füllstoffe, zugemischt werden.

Das mittels der Heißschmelzklebemasse selbstklebend ausgerüstete Trägermaterial weist weiter vorzugsweise beim Verkleben auf Stahl und auf der Trägerrückseite eine Klebkraft von mindestens 1,5 N/cm, besonders eine Klebkraft zwischen 2,5 N/cm und 5 N/cm, auf. Auf anderen Untergründen können höhere Klebkräfte erreicht werden.

insbesondere an medizinische Produkte, beispielsweise eine orthopädische Binde, werden hohe Anforderungen bezüglich der Klebeeigenschaften gestellt. Für eine ideale Anwendung sollte die Heißschmelzklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen der Lagen kommt, eine hohe Scherfestigkeit der Heißschmelzklebemasse notwendig.

Durch die gezielte Absenkung der Glasübergangstemperatur der Heißschmelzklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht.

Die hohe Scherfestigkeit der hier eingesetzten Heißschmelzklebemasse wird durch die hohe Kohäsivität des Blockcopolymeren erreicht. Die gute Anfaßklebrigkeit ergibt sich durch die eingesetzte Palette an Klebrigmachem und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Dazu wird insbesondere ein schubspannungsgesteuertes Rheometer verwendet. Derartige Rheometer sind bekannt.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur der Heißschmelzkleber zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G'' viskoser Anteil) und dem Speichermodul (G' elastischer Anteil) ermittelt. Für das subjektive Empfinden der Anfaßklebrigkeit (Tack) wird eine hohe Frequenz gewählt, sowie für die Scherfestigkeit eine niedrige Frequenz.

Eine hoher Zahlenwert bedeutet eine bessere Anfaßklebrigkeit und eine schlechtere Scherstabilität.

Der komplexe-dynamische Glasübergangspunkt ist der Übergangspunkt vom amorphen in den viskoelastischen Bereich. Er entspricht dem Maximum der Temperaturfunktion bei vorgegebener Frequenz.

$$Q = \tan \delta = G''/G'$$

| Bezeichnung | $T_G$ niedrige Frequenz | Scherstabilität niedrige Frequenz / RT | Anfaßklebrigkeit hohe Frequenz / RT |
|---|---|---|---|
| Heißschmelzkleber A | -12 ± 2 °C | tan δ = 0,18 ± 0,03 | tan δ = 0,84 ± 0,03 |
| Heißschmelzkleber B | -9 ± 2 °C | tan δ = 0,29 ± 0,05 | tan δ = 1,70 ± 0,10 |

Bevorzugt werden erfindungsgemäß Heißschmelzklebemassen, bei denen das Verhältnis des Verlustmoduls (viskoser Anteil) zum Speichermodul (elastischer Anteil) bei einer Frequenz von 100 rad/s bei 25 °C größer ist als 0,7, bevorzugt 0,9 bis 4,5, oder Heißschmelzklebemassen, bei denen das Verhältnis des viskosen Anteils zum elastischen Anteil bei einer Frequenz von 0,1 rad/s bei 25 °C kleiner ist als 0,40, bevorzugt zwischen 0,35 bis 0,02 und besonders bevorzugt zwischen 0,30 und 0,10.

Vorteilhaft insbesondere für die Verwendung bei medizinischen Produkten ist weiterhin, wenn die Heißschmelzklebemasse partiell auf dem Trägermaterial aufgetragen ist, beispielsweise durch Rasterdruck, Thermosiebdruck oder Tiefdruck, denn im Vollstrich selbstklebend ausgerüstete Trägermaterialien können bei der Applikation mechanische Hautirritationen hervorrufen.

Der partielle Auftrag ermöglicht durch geregelte Kanäle die Abführung des transepidermalen Wasserverlustes und verbessert das Ausdampfen der Haut beim Schwitzen, insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten auch unter Verwendung eines mehrlagigen Verbandes.

Erfolgt der partielle Auftrag der Heißschmelzklebemasse im Thermosiebdruck, so ist bei der Siebschablone das Steg/Loch-Verhältnis kleiner 2:1, bevorzugt kleiner oder gleich 1:1.
Das Prinzip des Thermosiebdrucks besteht in der Verwendung einer rotierenden beheizten, nahtlosen, trommelförmigen perforierten Rundschablone, die über eine Düse mit der Heißschmelzklebemasse beschickt wird. Eine speziell geformte Düsenlippe (Rund- oder Vierkantrakel) preßt die über einen Kanal eingespeiste Selbstklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Bevorzugt wird, wie bereits oben ausgeführt, der Auftrag in Form von Kalotten und ganz besonders solche Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Die Ausbildung der kleinen Klebstoffkalotten geschieht dabei nach folgendem Mechanismus:

Der Düsenrakeldruck fördert die Selbstklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haftenden Basis der Kalotten der in den Löchern begrenzte Vorrat an Heißschmelzklebemasse konturenscharf abgezogen bzw. durch den Rakeldruck auf den Träger gefördert.
Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie z.B. Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.
Die Positionierung der Kalotten auf dem Träger wird durch die in weiten Grenzen variierbare Geometrie des Auftragswerkes, zum Beispiel die Gravur- oder die Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 100 m/min, beschichtet, wobei die Beschichtungstemperatur größer als die Erweichungstemperatur zu wählen ist.

Femer kann die Heißschmelzklebemasse beispielsweise auch aufgesprüht sein, was ein mehr oder weniger unregelmäßiges Auftragsbild ergibt.

Die Heißschmelzklebemasse kann gleichmäßig auf dem Trägermaterial verteilt sein, sie kann aber auch funktionsgerecht für das Produkt über die Fläche unterschiedlich stark oder dicht aufgetragen sein.

Die Kombination der Heißschmelzklebemasse und der partiellen Beschichtung sichert auf der einen Seite eine sichere Verklebung des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert. Die Verankerung der Heißschmelzklebemasse auf dem Trägermaterial ist zudem mit bis zu 12 N/cm (Probenbreite) für medizinische Anwendungen gut. Durch die ausgeformten Sollbruchstellen in der partiellen Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft dieser selbstklebend ausgerüsteten Trägermaterialien aufweist, die Ablösbarkeit. Der angelegte Verband zeigt gute propriorezeptive Wirkungen.

Je nach der Ausstattung des Trägermaterials und dessen Temperaturempfindlichkeit kann die Heißschmelzklebeschicht direkt aufgetragen sein oder zuerst auf einen Hilfsträger aufgebracht und dann auf den endgültigen Träger transferiert werden. Auch ein nachträgliches Kalandem des beschichteten Produktes und/oder eine Vorbehandlung des Trägers, wie Coronabestrahlung, zur besseren Verankerung der Klebeschicht kann vorteilhaft sein.

Das erfindungsgemäße, selbstklebend ausgerüstete Trägermaterial wird für medizinische Anwendungsfälle weiter vorzugsweise nach der Applikation eingedeckt oder mit einer Wundauflage oder Polsterung versehen.

Darüber hinaus kann das selbstklebend ausgerüstete Trägermaterial sterilisierbar, bevorzugt γ (gamma)-sterilisierbar, sein.

Die hervorragenden Eigenschaften des erfindungsgemäßen, selbstklebend ausgerüsteten Trägermaterials legen die Verwendung für medizinische Produkte, insbesondere Pflaster, medizinische Fixierungen, Wundabdeckungen, orthopädische oder phlebologische Bandagen und Binden nahe.

Vorteilhaft zeigte sich, daß ein so selbstklebend beschichtetes, verstärktes Trägermaterial beim Durchnässen, wie es zum Beispiel bei wassersportlichen Aktivitäten unvermeidlich ist, eine gegenüber den handelsüblichen Trägermaterialien verbesserte Stabilität. Die relative Zunahme der Höchstzugkraft-Dehnung erfindungsgemäßer selbstklebend ausgerüsteter Trägermaterialien ist nach dem Durchnässen nur halb so groß wie bei handelsüblichen selbstklebend ausgerüsteten Trägermaterialien.

Hierdurch werden die erfindungsgemäßen Trägermaterialien, die ja somit im wesentlichen unelastisch sind, für spezielle medizinische Zwecke nutzbar, und weiter können Trägermaterialien, deren Einsatz bisher an mangelnder Festigkeit und/oder zu hoher Dehnung gescheitert ist, eingesetzt werden.

Bevorzugt können Trägermaterialien auf Basis von Gewebe, Gewirke, Vliese oder Verbundprodukte verwendet werden, sofern sie ansonsten die Anforderungen an den medizinischen Einsatz erfüllen.


**Beispiel**


Im folgenden sei beispielhaft ein bevorzugtes Trägermaterial beschrieben, ohne damit die Erfindung unnötig einschränken zu wollen.

Die Fadenkonstellation des Trägermaterials war 30 Fäden in Kettrichtung und 28 Fäden in Längsrichtung. Jeder 12te Kettfaden wurde durch ein Verstärkungsfaden aus Glasfaser ersetzt. Die übrigen Kett- und Schußfäden bestanden aus Baumwolle.

Die Fäden wurden dabei so ausgewählt, daß sie sich nicht signifikant in der Dicke unterscheiden.

Die Herstellung solcher Trägermaterialien ist technisch bekannt und kann beispielsweise unter Verwendung von zwei Kettbäumen oder durch Zulaufenlassen der Verstärkungsfäden aus einem Gatter erfolgen. Nachfolgend kann das Trägermaterial über verschiedenste Schritte ausgerüstet werden. Beispielhaft sind Textiltechnologien wie das Entschlichten, Sengen, Kalandern, Recken, Färben, Hydrophobieren usw. genannt.

Das so hergestellte Trägermaterial wies in Längsrichtung eine Höchstzugkraft von ca. 90 N/cm und eine Höchstzugkraft-Dehnung von 8% auf. Das Flächengewicht betrug ca. 120 g/cm$^2$. Nach dem vollständigen Durchnässen wies das Trägermaterial eine Höchstzugkraft von 85 N/cm auf. Die Höchstzugkraft-Dehnung betrug 12%.

Das Trägermaterial wies eine Luftdurchlässigkeit von 100 cm$^3$/(cm$^2$*s) und Wasserdampfdurchlässigkeit von größer 2500 g/(m$^2$*24h) auf und ließ sich von Hand in Querrichtung und Längsrichtung ein- und durchreißen.

Insgesamt ließ sich das Trägermaterial im durchnäßten Zustand weniger stark dehnen als vergleichbare Träger, welche nur aus Baumwolle bestanden.

Die Heißschmelzklebemasse setzte sich wie folgt zusammen:

- ein A-B/A-B-A Blockcopolymer, welches aus harten und weichen Segmenten besteht, mit einem Verhältnis der A-B-A zur A-B von 2:1 und einem Styrolgehalt im Polymer von 13 Mol.-%; der Anteil an der Klebemasse beträgt 40 Gew.-% (Kraton G),
- ein Paraffinkohlenwasserstoffharz mit einem Anteil an der Klebmasse von 55 Gew.-%,
- Kohlenwasserstoffharze mit einem Anteil von 4,5 Gew.-% (Super Resin HC 140),

- ein Alterungsschutzmittel mit einem Anteil von weniger als 0,5 Gew.-% (Irganox 1010).

Die eingesetzten Komponenten wurden in einem Thermomischer bei 175 °C homogenisiert.
Der Erweichungspunkt dieser Klebemasse betrug ca. 85 °C (DIN 52011), und die Klebmasse zeigte eine Viskosität von 2100 mPas bei 175 °C (DIN 53018, Brookfield DV II, Sp. 21). Die Glasübergang betrug nach oben genannter Methode -9 °C.
Die Heißschmelzklebemasse wurde im Thermosiebdruck auf den Träger appliziert.
Die direkte Beschichtung erfolgte mit 50 m/min bei einer Temperatur von 120 °C. Das Trägermaterial wurde mit 120 g/m$^2$ partiell beschichtet, wobei eine 14 Meshsieb-Schablone mit einer Siebstärke von 300 µm verwendet wurde.
Die nach diesem Verfahren hergestellte Binde zeigte ein reversibles Ablösen von der Haut sowie eine gute Luft- und Wasserdampfdurchlässigkeit. Aufgrund der hohen Scherstabilität des Schmelzhaftklebers wurde eine ausreichend Stabilisierung und eine gute propriorezeptive Wirkung festgestellt. Es wurden keine Hautirritationen und eine vernachlässigbar geringe Epilation nach dem Abnehmen der Binde beobachtet.

## Patentansprüche

1. Unelastisches Trägermaterial für medizinische Zwecke enthaltend einen Zusatz von hochfesten Fasern, Zwirnen, Mischzwimen oder Fäden, die eine Höchstzugkraft von mindestens 60 cN/tex und eine Wasseraufnahme von weniger als 10% zeigen und dass das Trägermaterial mindestens einseitig mit einer Heißschmelzklebemasse zumindest partiell beschichtet ist, **dadurch gekennzeichnet, dass** die Fasern, Zwirnen, Mischzwimen oder Fäden dem Trägermaterial eine Höchstzugkraft von mindestens 50 N/cm verleihen und die Heißschmelzklebemasse eine dynamisch-komplexe Glasübergangstemperatur bei einer Frequenz von 0,1 rad/s von weniger als 5 °C aufweist.

2. Trägermaterial nach Anspruch 1, **dadurch gekennzeichnet, dass** das Trägermaterial einen Zusatz von hochfesten Fasern, Zwirnen, Mischzwirnen oder Fäden mit einer Höchstzugkraft von 80 bis 500 cN/tex aufweist.

3. Trägermaterial nach Anspruch 1 oder 2, **dadurch gekennzeichnet, dass** das Trägermaterial einen Zusatz von hochfesten Fasern, Zwirnen, Mischzwimen oder Fäden mit einer Wasseraufnahme von weniger als 5%, besonders bevorzugt weniger als 3% zeigen.

4. Trägermaterial nach Anspruch 1, 2 oder 3, **dadurch gekennzeichnet, dass** die hochfesten Fasern, Zwirnen, Mischzwirnen oder Fäden dem Trägermaterial eine Höchstzugkraft von 60 bis 450 N/cm, besonders bevorzugt 65 bis 250 N/cm verleihen.

5. Trägermaterial für medizinische Zwecke nach einem der vorstehenden Ansprüche, **dadurch gekennzeichnet, daß** das Trägermaterial eine Dehnung von weniger als 10% bei einer Belastung von 10 N/cm aufweist.

6. Trägermaterial für medizinische Zwecke nach einem der vorstehenden Ansprüche, **dadurch gekennzeichnet, daß** das Trägermaterial ein Flächengewicht von weniger als 350 g/m$^2$, bevorzugt weniger 200 g/m$^2$ ausweist.

7. Trägermaterial für medizinische Zwecke nach einem der vorstehenden Ansprüche, **dadurch gekennzeichnet, daß** das Trägermaterial eine Höchstzugkraftdehnung unter 25%, bevorzugt unter 15%, besonders bevorzugt unter 10%, aufweist.

8. Trägermaterial für medizinische Zwecke nach einem der vorstehenden Ansprüche, **dadurch gekennzeichnet, daß** das Trägermaterial mit einem Faden oder mehreren Fäden aus Monofil, Multifil, Stapelfasergam oder Spinnfasergam und/oder mit orientierten hochfesten Fasern verstärkt ist.

9. Trägermaterial für medizinische Zwecke nach einem der vorhergehenden Ansprüche, **dadurch gekennzeichnet, daß** das Trägermaterial mit den Fäden und/oder hochfesten Fasern laminiert ist.

10. Trägermaterial für medizinische Zwecke nach einem der vorhergehenden Ansprüche, **dadurch gekennzeichnet, daß** die Fäden und/oder hochfesten Fasern in das Trägermaterial eingearbeitet sind.

11. Trägermaterial für medizinische Zwecke nach einem der vorhergehenden Ansprüche, **dadurch gekennzeichnet, daß** die Fäden und/oder hochfesten Fasern in das Trägermaterial eingelassen sind.

**12.** Trägermaterial für medizinische Zwecke nach einem der vorhergehenden Ansprüche, **dadurch gekennzeichnet, daß** durch den Zusatz von hochfesten Fasern oder Fäden aus einem Material mit einer Höchstzugkraft über 60 cN/tex das Trägermaterial eine Höchstzugkraft über 65 N/cm und eine Höchstzugkraftdehnung unter 25% bei einem Flächengewicht von unter 140 g/m$^2$ hat.

**13.** Trägermaterial für medizinische Zwecke nach einem der vorhergehenden Ansprüche, **dadurch gekennzeichnet, daß** sich das Trägermaterial bei einer Belastung von 30N/cm nach 50 Zyklen weniger als 20%, bevorzugt weniger 10%, besonders bevorzugt weniger als 5%, verformen läßt.

**14.** Trägermaterial für medizinische Zwecke nach einem der vorhergehenden Ansprüche, **dadurch gekennzeichnet, daß** das Trägermaterial von Hand senkrecht zur Orientierung der Verstärkung und/oder in Richtung der Verstärkung reißbar ist.

**15.** Trägermaterial für medizinische Zwecke nach einem der vorhergehenden Ansprüche, **dadurch gekennzeichnet, daß** die Heißschmelzklebemasse eine dynamisch-komplexe Glasübergangstemperatur bei einer Frequenz von 0,1 rad/s von -6 °C bis -30 °C, besonders bevorzugt von -9 °C bis -25 °C, aufweist.

**16.** Trägermaterial für medizinische Zwecke nach einem der vorhergehenden Ansprüche, **dadurch gekennzeichnet, daß** das Verhältnis des Verlustmoduls (viskoser Anteil) zum Speichermodul (elastischer Anteil) der Heißschmelzklebemasse bei einer Frequenz von 100 rad/s bei 25 °C größer 0,7, bevorzugt 0,9 bis 4,5, ist.

**17.** Trägermaterial für medizinische Zwecke nach einem der vorhergehenden Ansprüche, **dadurch gekennzeichnet, daß** das Verhältnis des des Verlustmoduls (viskoser Anteil) zum Speichermodul (elastischer Anteil) der Heißschmelzsklebemasse bei einer Frequenz von 0,1 rad /s bei 25 °C kleiner 0,40, bevorzugt zwischen 0,35 bis 0,02, besonders bevorzugt zwischen 0,30 und 0,10, ist.

**18.** Trägermaterial für medizinische Zwecke nach einem der vorhergehenden Ansprüche, **dadurch gekennzeichnet, daß** die Heißschmelzklebemasse auf Blockcopolymerbasis aufgebaut ist, insbesondere A-B- oder A-B-A-Blockcopolymere oder deren Mischungen, wobei Phase A vornehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

**19.** Trägermaterial für medizinische Zwecke nach Anspruch 18, **dadurch gekennzeichnet, daß** der gesamte Styrolanteil im Polymer niedriger ist als 35 Gew.-%, bevorzugt 5 Gew.-% bis 30 Gew.-%.

**20.** Trägermaterial für medizinische Zwecke nach einem der Ansprüche 18 oder 19, **dadurch gekennzeichnet, daß** die Heißschmelzklebemasse besteht aus

- 10 Gew.-% bis 90 Gew.-% Blockcopolymeren,
- 5 Gew.-% bis 80 Gew.-% Klebrigmachem wie Ölen, Wachsen, Harzen und deren Mischungen, bevorzugt Mischungen aus Harzen und Ölen,
- weniger als 60 Gew.-% Weichmachern,
- weniger als 15 Gew.-% Additiven
- weniger als 5 Gew.-% Stabilisatoren.

**21.** Trägermaterial für medizinische Zwecke nach einem der Ansprüche 18 bis 20, **dadurch gekennzeichnet, daß** die Heißschmelzklebemasse durch Rasterdruck, Thermosiebdruck oder Tiefdruck aufgebracht ist.

**22.** Trägermaterial für medizinische Zwecke nach einem der Ansprüche 18 bis 21, **dadurch gekennzeichnet, daß** die Heißschmelzklebemasse in Form von polygeometrischen Kalotten auf das Trägermaterial gebracht wird.

**23.** Trägermaterial für medizinische Zwecke nach einem der Ansprüche 18 bis 22, **dadurch gekennzeichnet, daß** die Heißschmelzklebemasse mit einem Flächengewicht von größer 15 g/m$^2$, bevorzugt zwischen 70 g/m$^2$ und 300 g/m$^2$, ganz besonders bevorzugt zwischen 90 g/m$^2$ und 160 g/m$^2$, auf dem Trägermaterial beschichtet ist.

**24.** Trägermaterial für medizinische Zwecke nach einem der Ansprüche 18 bis 23, **dadurch gekennzeichnet, daß** das beschichtete Trägermaterial aufweist eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), und/oder eine Wasserdampfdurchlässigkeit von

größer 500 g/(m$^2$*24h), bevorzugt größer 1000 g/(m$^2$*24h), ganz besonders bevorzugt größer 2000 g/(m$^2$*24h).

25. Trägermaterial für medizinische Zwecke nach einem der Ansprüche 18 bis 24, **dadurch gekennzeichnet, daß** das selbstklebend ausgerüstete Trägermaterial nach der Applikation eingedeckt oder mit einer Wundauflage oder Polsterung versehen wird.

26. Trägermaterial für medizinische Zwecke nach einem der Ansprüche 18 bis 25, **dadurch gekennzeichnet, daß** das selbstklebend ausgerüstete Trägermaterial sterilisierbar, bevorzugt γ (gamma)-sterilisierbar, ist.

27. Verwendung eines selbstklebend ausgerüsteten Trägermaterials gemäß einem der vorgehenden Ansprüche für medizinische Produkte, insbesondere Pflaster, medizinische Fixierungen, Wundabdeckungen, orthopädische oder phlebologische Bandagen und Binden.

**Claims**

1. Inelastic backing material for medical purposes, comprising an addition of high-strength fibres, multi-strand yarns, mixed multistrands or filaments which exhibit an ultimate tensile stress strength of at least 60 cN/tex and a water absorption of less than 10% and that the backing material is coated at least partially on at least one side with a hot-melt adhesive composition, **characterized in that** the fibres, multi-strand yarns, mixed multistrands or filaments give the backing material an ultimate tensile stress strength of at least 50 N/cm and the hot-melt adhesive composition has a dynamic-complex glass transition temperature at a frequency of 0.1 rad/s of less than 5°C.

2. Backing material according to Claim 1, **characterized in that** the backing material has an addition of high-strength fibres, multi-strand yarns, mixed multistrands or filaments having an ultimate tensile stress strength of from 80 to 500 cN/tex.

3. Backing material according to Claim 1 or 2, **characterized in that** the backing material has an addition of high-strength fibres, multi-strand yarns, mixed multistrands or filaments having a water absorption of less than 5%, more preferably less than 3%.

4. Backing material according to Claim 1, 2 or 3, **characterized in that** the high-strength fibres, multi-strand yarns, mixed multistrands or filaments give the backing material an ultimate tensile stress strength of from 60 to 450 N/cm, more preferably from 65 to 250 N/cm.

5. Backing material for medical purposes according to any of the preceding claims, **characterized in that** the backing material has an elongation of less than 10% under a load of 10 N/cm.

6. Backing material for medical purposes according to any of the preceding claims, **characterized in that** the backing material has a basis weight of less than 350 g/m$^2$, preferably less than 200 g/m$^2$.

7. Backing material for medical purposes according to any of the preceding claims, **characterized in that** the backing material has an ultimate tensile stress elongation of less than 25%, preferably less than 15%, more preferably less than 10%.

8. Backing material for medical purposes according to any of the preceding claims, **characterized in that** the backing material has been reinforced with one or more monofil, multifil, staple fibre or spun fibre yarns and/or with oriented high-strength fibres.

9. Backing material for medical purposes according to any of the preceding claims, **characterized in that** the backing material is laminated with the filaments and/or high-strength fibres.

10. Backing material for medical purposes according to any of the preceding claims, **characterized in that** the filaments and/or high-strength fibres have been incorporated into the backing material.

11. Backing material for medical purposes according to any of the preceding claims, **characterized in that** the filaments and/or high-strength fibres have been embedded in the backing material.

**12.** Backing material for medical purposes according to any of the preceding claims, **characterized in that**, through the addition of high-strength fibres or filaments of a material having an ultimate tensile stress strength of more than 60 cN/tex, the backing material has an ultimate tensile stress strength of more than 65 N/cm and an ultimate tensile stress elongation of less than 25% at a basis weight of less than 140 g/m$^2$.

**13.** Backing material for medical purposes according to any of the preceding claims, **characterized in that** the backing material can be deformed by less than 20%, more preferably less than 10% and very preferably less than 5% after 50 cycles at a load of 30 N/cm.

**14.** Backing material for medical purposes according to any of the preceding claims, **characterized in that** the backing material can be torn by hand perpendicular to the orientation of the reinforcement and/or in the direction of the reinforcement.

**15.** Backing material for medical purposes according to any of the preceding claims, **characterized in that** the hot-melt adhesive composition has a dynamic-complex glass transition temperature at a frequency of 0.1 rad/s of from -6°C to -30°C, with particular preference from -9°C to -25°C.

**16.** Backing material for medical purposes according to any of the preceding claims, **characterized in that** the ratio of the loss modulus (viscous component) to the storage modulus (elastic component) of the hot-melt adhesive composition at a frequency of 100 rad/s at 25°C is greater than 0.7, preferably from 0.9 to 4.5.

**17.** Backing material for medical purposes according to any of the preceding claims, **characterized in that** the ratio of the of the [sic] loss modulus (viscous component) to the storage modulus (elastic component) of the hot-melt adhesive composition at a frequency of 0.1 rad/s at 25°C is less than 0.40, preferably between 0.35 and 0.02, more preferably between 0.30 and 0.10.

**18.** Backing material for medical purposes according to any of the preceding claims, **characterized in that** the hot-melt adhesive composition is based on block copolymers, especially A-B or A-B-A block copolymers or mixtures thereof, where phase A is principally polystyrene or its derivatives and phase B is ethylene, propylene, butylene, butadiene, isoprene or mixtures thereof, particular preference being given here to ethylene and butylene or mixtures thereof.

**19.** Backing material for medical purposes according to Claim 18, **characterized in that** the overall styrene fraction in the polymer is less than 35% by weight, preferably from 5% by weight to 30% by weight.

**20.** Backing material for medical purposes according to either of Claims 18 and 19, **characterized in that** the hot-melt adhesive composition consists of

- from 10% by weight to 90% by weight of block copolymers,
- from 5% by weight to 80% by weight of tackifiers such as oils, waxes, resins and mixtures thereof, preferably mixtures of resins and oils,
- less than 60% by weight of plasticizers,
- less than 15% by weight of additives, and
- less than 5% by weight of stabilizers.

**21.** Backing material for medical purposes according to any of Claims 18 to 20, **characterized in that** the hot-melt adhesive composition is applied by halftone printing, thermal screen printing or intaglio printing.

**22.** Backing material for medical purposes according to any of Claims 18 to 21, **characterized in that** the hot-melt adhesive composition is applied in the form of polygeometric domes to the backing material.

**23.** Backing material for medical purposes according to any of Claims 18 to 22, **characterized in that** the hot-melt adhesive composition is coated on the backing material with a weight per unit area of greater than 15 g/m$^2$, preferably between 70 g/m$^2$ and 300 g/m$^2$, with very particular preference between 90 g/m$^2$ and 160 g/m$^2$.

**24.** Backing material for medical purposes according to any of Claims 18 to 23, **characterized in that** the coated backing material has an air permeability of greater than 1 cm$^3$/(cm$^2$*s), preferably greater than 15 cm$^3$/(cm$^2$*s), with very particular preference greater than 70 cm$^3$/(cm$^2$*s) and/or a water vapour permeability of greater than

500 g/(m$^2$*24h), preferably greater than 1000 g/(m$^2$*24h), with very particular preference greater than 2000 g/(m$^2$*24h).

25. Backing material for medical purposes according to any of Claims 18 to 24, **characterized in that** the self-adhesively equipped backing material is covered after application or is provided with a wound pad or with padding.

26. Backing material for medical purposes according to any of Claims 18 to 25, **characterized in that** the self-adhesively equipped backing material can be sterilized, preferably with γ (gamma) radiation.

27. Use of a self-adhesively equipped backing material according to any of the preceding claims for medical products, especially plasters, medical fixings, wound covers, orthopaedic or phlebological bandages and dressings.

**Revendications**

1. Matériau support inélastique destiné à des fins médicales contenant une addition de fibres, de fils retors, de fils retors mixtes ou de fils hautement résistants, qui présentent une force de traction maximale d'au moins 60 cN/tex, et une absorption d'eau inférieure à 10% et le matériau support est revêtu au moins partiellement au moins d'un côté par une masse autoadhésive fusible à chaud, **caractérisé en ce que** les fibres, fils retors, fils retors mixtes ou fibres confèrent au matériau support une force de traction maximale d'au moins 50 N/cm et la masse autoadhésive fusible à chaud présente une température de transition vitreuse complexe dynamique à une fréquence de 0,1 rad/s inférieure à 5°C.

2. Matériau support selon la revendication 1, **caractérisé en ce que** le matériau support présente une addition de fibres, de fils retors, de fils retors mixtes ou de fils hautement résistants, qui présentent une force de traction maximale de 80 à 500 cN/tex.

3. Matériau support selon les revendications 1 ou 2, **caractérisé en ce que** le matériau support présente une addition de fibres, de fils retors, de fils retors mixtes ou de fils hautement résistants présentant une absorption d'eau inférieure à 5%, de manière particulièrement préférée inférieure à 3%.

4. Matériau support selon les revendications 1, 2 ou 3, **caractérisé en ce que** les fibres, fils retors, fils retors mixtes ou fibres hautement résistants confèrent au matériau support une force de traction maximale de 60 à 450 N/cm, de manière particulièrement préférée de 65 à 250 N/cm.

5. Matériau support destiné à des fins médicales selon l'une quelconque des revendications précédentes, **caractérisé en ce que** le matériau support présente un allongement inférieur à 10% à une charge de 10 N/cm.

6. Matériau support destiné à des fins médicales selon l'une quelconque des revendications précédentes, **caractérisé en ce que** le matériau support présente un poids surfacique inférieur à 350 g/m$^2$, de préférence inférieur à 200 g/m$^2$.

7. Matériau support destiné à des fins médicales selon l'une quelconque des revendications précédentes, **caractérisé en ce que** le matériau support présente un allongement à la force de traction maximale inférieur à 25%, de préférence inférieur à 15%, de manière particulièrement préférée inférieur à 10%.

8. Matériau support destiné à des fins médicales selon l'une quelconque des revendications précédentes, **caractérisé en ce que** le matériau support est renforcé par un ou plusieurs fils en monofil, en multifil, en fibres discontinues ou en fibres filées et/ou par des fibres orientées hautement résistantes.

9. Matériau support destiné à des fins médicales selon l'une quelconque des revendications précédentes, **caractérisé en ce que** le matériau support est stratifié avec les fils et/ou les fibres hautement résistantes.

10. Matériau support destiné à des fins médicales selon l'une quelconque des revendications précédentes, **caractérisé en ce que** les fils et/ou les fibres hautement résistantes sont incorporés dans le matériau support.

11. Matériau support destiné à des fins médicales selon l'une quelconque des revendications précédentes, **caractérisé en ce que** les fils et/ou les fibres hautement résistantes sont encastrés dans le matériau support.

**12.** Matériau support destiné à des fins médicales selon l'une quelconque des revendications précédentes, **caractérisé en ce que** le matériau support présente une force de traction maximale supérieure à 65 N/cm et un allongement à la force de traction maximale inférieur à 25% à un poids surfacique inférieur à 140 g/m$^2$ par l'addition de fibres ou de fils hautement résistants en un matériau présentant une force de traction maximale supérieure à 60 cN/tex.

**13.** Matériau support destiné à des fins médicales selon l'une quelconque des revendications précédentes, **caractérisé en ce que** le matériau support est déformé à raison de moins de 20%, de préférence de moins de 10%, de manière particulièrement préférée de moins de 5% lors d'une charge de 30 N/cm après 50 cycles.

**14.** Matériau support destiné à des fins médicales selon l'une quelconque des revendications précédentes, **caractérisé en ce que** le matériau de support peut être déchiré à la main perpendiculairement par rapport à l'orientation du renforcement et/ou dans le sens du renforcement.

**15.** Matériau support destiné à des fins médicales selon l'une quelconque des revendications précédentes, **caractérisé en ce que** la masse autoadhésive fusible à chaud présente une température de transition vitreuse complexe dynamique de -6°C à -30°C, de manière particulièrement préférée de -9°C à -25°C, à une fréquence de 0,1 rad/s.

**16.** Matériau support destiné à des fins médicales selon l'une quelconque des revendications précédentes, **caractérisé en ce que** le rapport module de perte (proportion visqueuse) à module d'accumulation (proportion élastique) de la masse autoadhésive fusible à chaud, à une fréquence de 100 rads/s à 25°C, est supérieur à 0,7, de préférence de 0,9 à 4,5.

**17.** Matériau support destiné à des fins médicales selon l'une quelconque des revendications précédentes, **caractérisé en ce que** le rapport module de perte (proportion visqueuse) à module d'accumulation (proportion élastique) de la masse autoadhésive fusible à chaud, à une fréquence de 0,1 rad/s à 25°C, est inférieur à 0,40, de préférence entre 0,35 à 0,02, de manière particulièrement préférée entre 0,30 et 0,10.

**18.** Matériau support destiné à des fins médicales selon l'une quelconque des revendications précédentes, **caractérisé en ce que** la masse autoadhésive fusible à chaud est réalisée à base de copolymères à blocs, en particulier de copolymères à blocs A-B ou A-B-A ou leurs mélanges, la phase A étant principalement du polystyrène ou ses dérivés et la phase B de l'éthylène, du propylène, du butylène, du butadiène, de l'isoprène ou leurs mélanges, de manière particulièrement préférée l'éthylène et le butylène ou leurs mélanges.

**19.** Matériau support destiné à des fins médicales selon la revendication 18, **caractérisé en ce que** la proportion totale en styrène dans le polymère est inférieure à 35% en poids, de préférence de 5% en poids à 30% en poids.

**20.** Matériau support destiné à des fins médicales selon l'une quelconque des revendications 18 ou 19, **caractérisé en ce que** la masse autoadhésive fusible à chaud est constituée de

- 10% en poids à 90% en poids de copolymères à blocs,
- 5% en poids à 80% en poids d'adhésifs tels que des huiles, des cires, des résines et leurs mélanges, de préférence des mélanges de résines et d'huiles,
- moins de 60% en poids de plastifiants,
- moins de 15% en poids d'additifs,
- moins de 5% en poids en poids de stabilisateurs.

**21.** Matériau support destiné à des fins médicales selon l'une quelconque des revendications 18 à 20, **caractérisé en ce que** la masse autoadhésive fusible à chaud est appliquée par impression matricielle, par thermosérigraphie ou par héliogravure.

**22.** Matériau support destiné à des fins médicales selon l'une quelconque des revendications 18 à 21, **caractérisé en ce que** la masse autoadhésive fusible à chaud est appliquée sous forme de calottes poly-géométriques sur le matériau support.

**23.** Matériau support destiné à des fins médicales selon l'une quelconque des revendications 18 à 22, **caractérisé en ce que** la masse autoadhésive fusible à chaud est revêtue sur le matériau support à un poids surfacique supérieur à 15 g/m$^2$, de préférence entre 70 g/m$^2$ et 300 g/m$^2$, de manière particulièrement préférée entre 90 g/

$m^2$ et 160 g/m$^2$.

**24.** Matériau support destiné à des fins médicales selon l'une quelconque des revendications 18 à 23, **caractérisé en ce que** le matériau support revêtu présente une perméabilité à l'air supérieure à 1 cm$^3$/(cm$^2$*s), de préférence supérieure à 15 cm$^3$/(cm$^2$*s), de manière particulièrement préférée supérieure à 70 cm$^3$/(cm$^2$*s) et/ou une perméabilité à la vapeur d'eau supérieure à 500 g/(m$^2$*24 h), de préférence supérieure à 1000 g/(m$^2$*24 h), de manière particulièrement préférée supérieure à 2000 g/(m$^2$*24 h).

**25.** Matériau support destiné à des fins médicales selon l'une quelconque des revendications 18 à 24, **caractérisé en ce que** le matériau support apprêté de manière autoadhésive est recouvert après l'application ou pourvu d'un tissu chirurgical ou d'un rembourrage.

**26.** Matériau support destiné à des fins médicales selon l'une quelconque des revendications 18 à 25, **caractérisé en ce que** le matériau support apprêté de manière autoadhésive peut être stérilisé, de préférence par une stérilisation γ (gamma).

**27.** Utilisation d'un matériau support apprêté de manière autoadhésive selon l'une quelconque des revendications précédentes pour des produits médicaux, en particulier des pansements, des fixations médicales, des recouvrements chirurgicaux, des bandages et des bandes orthopédiques ou en cas de phlébites.

a)

b)

c)

d)

Figur 1